# EUROPEAN PATENT SPECIFICATION

(11) **EP 2 285 269 B1**
(45) Date of publication and mention of the grant of the patent: **07.11.2012**
(21) Application number: 09738574.4
(22) Date of filing: 30.04.2009
(51) Int. Cl.: A61M 16/06, A61B 5/00, A61B 5/083

(54) **WIRELESS CAPNOGRAPHY**
DRAHTLOSE KAPNOGRAPHIE
CAPNOGRAPHIE SANS FIL

(30) Priority: 29.04.2008 US 71438 P
(43) Date of publication of application: 23.02.2011
(73) Proprietor: Oridion Medical 1987 Ltd., 91450 Jerusalem (IL)
(72) Inventor: COLMAN, Joshua Lewis, 97430 Jerusalem (IL); BOTESAZAN, Zion, 97704 Jerusalem (IL); DAVID, Uri, 70400 Ness Ziona (IL)
(74) Representative: Becker Kurig Straus
(86) International application number: PCT/IL2009/000457
(87) International publication number: WO 2009/133561

(56) References cited:
- WO-A-01/95971
- WO-A-2007/140478
- US-A1- 2005 171 444
- US-B1- 6 921 369

## Description

### BACKGROUND

Medical monitoring systems that are routinely used in various health care settings, to track and monitor various patient related parameters may generally and arbitrarily be divided into two main functional units: a patient interface unit and a control unit. Those two main units interconnect with each other in order for the medical monitoring system to function and provide measurements of the medical parameters being sensed/measured/monitored. The connection between the units of the monitoring system generally requires the use of various wires and/or tubes that physically connect the units. However, the use of wires and/or tubes may have several disadvantages. Some of the disadvantages may include, for example: 1. Physical connections (such as by wires, cables or tubes) between the patient and the medical monitoring systems that monitor the patient status may interfere with the patient comfort. 2. In critical care settings, the patient may often be moved and the patient movement may cause misplacement, kinking or disconnection of the patient from the monitoring system. 3. Health care providers require the ability to reach the patient from all directions, and hence physical connections (such as, for example by tubes, tubing connections, wires, wiring and the like), may interfere with the accessibility of the health care provider to the patient. 4. Patients in critical care may often be transported to different locations to perform various testing and procedures, such as, for example, x-rays, surgery and the like. Disconnecting and reconnecting patients from bedside medical monitoring system to transportable monitoring systems may cause mistakes, errors and the like and further dictates similarity between the transportable and bedside monitors, in order to minimize such errors. 5. During transport and emergency, wires and tubing are vulnerable to kinking, pulling out, misplacing, breakage and the like. In addition, such wires and tubing may further interfere (such as by causing tripping, and the like during transport). 6. Patient's mobility considerations may be particularly relevant in pain management (as opposed to critical care). The patient may often desire to leave his bed, a task which may be impeded by the wires and tubes which connect the patient to the monitoring systems. 7. Usually, a patient interface with several separate monitoring systems that cannot all be kept at the same position relatively to the patient. This may lead to the creation of a web of wires/tubes/lines in different direction. 8. In many cases, an optimal position for the monitoring system (with regards of ease of operation by the health care provider) may not be appropriate with respect to the wires/tubes connection to the patient. There is thus a need in the art for improved medical monitoring systems and improved functional connection of these systems to the patient.

The invention is defined in the independent claims 1 and 11, which are partitioned relative to the document WO 2007/140978 A.

The following embodiments and aspects thereof are described and illustrated in conjunction with systems, tools and methods which are meant to be exemplary and illustrative, not limiting in scope. In various embodiments, one or more of the above-described problems have been reduced or eliminated, while other embodiments are directed to other advantages or improvements.

According to some embodiments, there is provided a medical monitoring system which includes more than one separable functional unit, wherein the connection between at least some of the functional units is wireless and/or tubeless. The medical monitoring system may be completely wireless and/or tubeless if none of the separable functional units are physically connected. The medical monitoring system may be at least partially wireless and/or tubeless if at least some of the separable functional units are not physically connected. The medical monitoring system may include various separable, functional units such as, for example, a patient interface unit, adapted to sample/sense/measure/collect physiological parameters related to a patient. The patient interface is functionally connected to a platform unit that is adapted to receive input from the patient interface and optionally from additional patient interfaces/devices and to transmit some or all of the information, preferably by wireless route to a remote main analyzing unit (station). The remote main analyzing unit is adapted to receive the information, process/analyze the information and present the analyzed/raw information on a display panel. The main analyzing unit may be further adapted to transmit said information to a remote location, such as, for example, a nurse station.

According to some exemplary embodiments, the wireless monitoring system may include a wireless capnography device. The patient interface may include a breath sampler, adapted to sample exhaled breath of a patient, for example, by use of a cannula. The platform unit may receive the sample of exhaled breath and sense within the sample the levels of CO₂. The data may be wirelessly transferred to the main analyzing unit that may further analyze the data and display the analyzed data, such as, for example, CO₂ concentration in exhaled breath (optionally with additional data, obtained from the measurements, such as, for example, the CO₂ waveform, changes in CO₂ levels in exhaled breath over time, and the like). The main analyzing unit may further include a patient interface that may allow the user (such as the health care provider) to determine operation parameters and other related parameters of the wireless capnography system.

According to some embodiments, there is thus provided a medical monitoring system that includes separable functional units, such as, for example, a patient interface, adapted to sample a physiological parameter of a patient; a platform unit, adapted to receive sample from said patient interface and sense a health related parameter within said sample; and main analyzing unit adapted to wirelessly receive information from said platform unit, analyze said information and display parameters related to said information, wherein the physical connection between at least two of the functional units is wireless and/or tubeless. The physiological parameter of the patient sampled by the patient interface may include, for example, exhaled breath. The health related parameter sensed by the platform unit may include a level of CO₂.

According to other embodiments, there is provided a medical monitoring system that includes more than one separable functional units, such as, a patient interface, adapted to sense a physiological parameter of a patient; a platform unit, adapted to receive information from said patient interface and sense a health related parameter within said sample; and main analyzing unit adapted to wirelessly receive information from said platform unit, analyze said information and display parameters related to said information, wherein the connection between at least two of the functional units is wireless and/or tubeless. The physiological parameter of the patient sensed by the patient interface may include, for example, blood pressure, heart rate, blood oxygen saturation levels, brain activity, heart electrical activity, temperature, pulmonary related parameters, breath rate, and the like or any combination thereof.

According to other embodiments, there is further provided a method for using a medical monitoring device, wherein the device functions at least partially by wireless and/or tubeless routes.

According to some exemplary embodiments, there is provided a wireless/tubeless capnography system that may be used to monitor patient's CO₂ levels in exhaled breath without the use of fluid tubing and other wiring to connect between the patient interface, which includes a sampling unit and a remote main unit.

According to other embodiments, there is provided a fluid analyzing system that includes a wireless and tubeless patient interface that includes a sensor adapted to sense fluids of a patient, said patient interface further includes: a data converter adapted to convert data obtained by the sensor to a digital data, a transmitter, adapted to transmit the digital data from the converter to a remote main unit. The remote main unit may include a receiver that is adapted to receive data transmitted from the sampling unit. The system may further include one or more portable power source(s) located in the patient interface. Alternatively, or in addition, the patient interface may further include a miniature pump, which is adapted to transfer at least a sample of a patient's breath towards a fluid sensor. The patient interface may further include a cannula, a breath guide, or any combination thereof, adapted to direct at least a sample of the exhaled breath towards a sampling area.

According to additional embodiments, the fluid analyzing device may include a capnograph adapted to detect (and optionally analyze and/or monitor) CO₂ levels in exhaled breath. According to some embodiments, the sampling unit may include a fluid sensor, such as a CO₂ sensor. The CO₂ sensor may include an IR sensor, a semiconductive type sensor, or any combination thereof.

According to additional embodiments, there is provided a method for conducting wireless/tubeless capnography, the method includes sensing CO₂ levels in exhaled breath by a CO₂ sensor, converting the data obtained by the CO₂ sensor to digital data by a converter, transmitting said digital data by a transmitter to a remote main unit that includes a receiver, adapted to receive the digital data. The remote main unit may further include one or more analyzers, one or more processors, or any combination thereof, that may be used to analyze, compute and/or calculate the data received from the sampling unit.

According to additional embodiments, the wireless/tubeless fluid analyzing system may be used in system and method with additional at least partially wireless and/or tubeless monitoring systems. According to some embodiments, there is provided a system and method for combined wireless and/or tubeless capnograph, wireless and/or tubeless pulse oximetry and wireless and/or tubeless respiratory rate measurement. According to some embodiments, there is provided a monitoring system and method for combined wireless and/or tubeless capnography and oxygen saturation measurements. According to additional embodiments, there is provided a monitoring system and method for combined wireless and/or tubeless capnography and non-invasive blood pressure measurements.

According to some embodiments, there is provided a breath monitoring device which includes a patient interface unit, adapted to be positioned on the patient head, said patient interface unit includes: i. a cannula, adapted to collect exhaled breath from a patient; ii. a pump adapted to transfer at least a sample of the collected exhaled breath from the cannula to a sampling area; iii. a CO₂ sensor located in or in proximity to the sampling area; iv. a control logic adapted to compute at least one CO₂ related parameter in the exhaled breath; and v. a transmitter adapted to wirelessly transmit data corresponding to the at least one CO₂ related parameter to a remote unit. The device may further include a tube connecting between the cannula and the sampling area.

According to further embodiments, the patient interface unit may further include one or more power sources, one or more data converters adapted to convert data obtained from the CO₂ sensor into digital data, or a combination thereof. The pump may include a miniature pump, low powered pump, low flow rate pump, or any combination thereof. The device may further include a carrying unit adapted to position the patient interface on the patient head.

According to additional embodiments, the cannula may be adapted to direct the exhaled breath from the mouth of the patient, from one or two nostrils of the patient, or a combination thereof.

According to yet further embodiments, the at least one CO₂ related parameter includes CO₂ concentration, CO₂ waveform, change in CO₂ concentration over time, End Tidal CO₂ (Et CO₂) or any combination thereof. According to additional embodiments, the patient interface unit may further include one or more additional sensors. The one or more additional sensors may include thermometer, blood oxygen saturation sensor, blood pressure sensor, heart rate sensor, respiration rate sensor, heart electrical activity sensor, brain activity sensor, or any combination thereof.

According to some embodiments, there is provided a breath monitoring device which includes a patient interface unit, adapted to be positioned on the patient head, said patient interface unit includes: i. a breathing guide, adapted to direct exhaled breath from a nostril and/or mouth of the patient towards a sampling area located in or in proximity to said breathing guide; ii. one or more CO₂ sensors located in the sampling area; iii. a control logic adapted to compute at least one CO₂ related parameter in the exhaled breath; and iv. a transmitter adapted to wirelessly transmit data corresponding to the at least one CO₂ related parameter to a remote unit. The one or more CO₂ sensors may include an infra red based CO₂ sensor, semi-conductor based CO₂ sensor, nanotechnology based CO₂ sensor, or any combination thereof.

According to additional embodiments, the patient interface unit may further include one or more power sources, one or more data converters adapted to convert data obtained from the CO₂ sensor into digital data, or a combination thereof. According to further embodiments, the device may further include a carrying unit adapted to position the patient interface on the patient head. According to additional embodiments, the at least one CO₂ related parameter may include CO₂ concentration, CO₂ waveform, change in CO₂ concentration over time, End Tidal CO₂ (Et CO₂) or any combination thereof.

According to additional embodiments, the patient interface unit further may further include one or more additional sensors. The one or more additional sensors may include: temperature sensor, blood oxygen saturation sensor, blood pressure sensor, heart rate sensor, respiration rate sensor, heart electrical activity sensor, brain activity sensor, or any combination thereof.

According to some embodiments, there is provided a breath monitoring device which includes: a patient interface unit, adapted to be positioned on the patient head, said patient interface unit comprises a cannula, adapted to collect a sample of exhaled breath from a patient; and a platform unit which includes: i. one or more CO₂ sensors adapted to sense CO₂ levels in the sample; ii. a pump adapted to transfer the sample from the cannula to the one or more CO₂ sensors; and iii. a communication unit adapted to wirelessly transmit data corresponding to the CO₂ levels to a remote analyzing unit.

According to additional embodiments, the platform unit may further include a control center, one or more power sources, one or more adapters, one or more additional sensors, or any combination thereof. The one or more additional sensors may include temperature sensor, blood oxygen saturation sensor, blood pressure sensor, heart rate sensor, respiration rate sensor, heart electrical activity sensor, brain activity sensor, or any combination thereof. The adapters may include adapters for connection to a monitoring device, sampler, O₂ supply, power source, or any combination thereof. According to further embodiments, the platform unit may be adapted to regulate the O₂ supply, regulation of the O₂ supply is such that the O₂ supply is stopped or reduced during the patient's exhalation.

According to yet further embodiments, the platform unit may be portable. The platform unit may be adapted to be connected with the patient's bed or chair. According to additional embodiments, the communication unit is further adapted to wirelessly receive information and/or data from the analyzing unit.

According to yet further embodiments, the data corresponding to the CO₂ levels may include concentration, CO₂ waveform, change in CO₂ concentration over time, End Tidal CO₂ (Et CO₂) or any combination thereof. According to further embodiments, the one or more CO₂ sensors may include an infra red based CO₂ sensor, semi-conductor based CO₂ sensor, nanotechnology based CO₂ sensor, or any combination thereof.

According to some embodiments there is provided a breath monitoring system which includes: a patient interface unit, adapted to be positioned on the patient head, said patient interface unit comprises: i. a cannula, adapted to collect exhaled breath from a patient; ii. a pump adapted to transfer at least a sample of the collected exhaled breath from the cannula to a sampling area; iii. a CO₂ sensor located in or in proximity to the sampling area; iv. a control logic adapted to compute at least one CO₂ related parameter in the exhaled breath; and v. a transmitter adapted to wirelessly transmit data corresponding to the CO₂ related parameters to a remote unit; and; a remote unit adapted to wirelessly receive from the patient interface unit the data corresponding to the at least one CO₂ related parameter and to further process and/or display said data or information related to said data. The system may further include a tube connecting between the cannula and the sampling area.

According to additional embodiments, the patient interface unit of the system may further include one or more power sources, one or more data converters adapted to convert data obtained from the CO₂ sensor into digital data, or a combination thereof.

According to yet additional embodiments, the pump of the system may include miniature pump, low powered pump, low flow rate pump, or any combination thereof. According to yet further embodiments, the system may further include a carrying unit adapted to position the patient interface on the patient head. According to additional embodiments the cannula of the system may be adapted to direct the exhaled breath from the mouth of the patient, from one or two nostrils of the patient, or any combination thereof.

According to yet additional embodiments, the at least one CO₂ related parameter comprises CO₂ concentration, CO₂ waveform, change in CO₂ concentration over time, End Tidal CO₂ (Et CO₂) or any combination thereof.

According to further embodiments, the patient interface of the system may further include one or more additional sensors. The one or more additional sensors comprise: thermometer, blood oxygen saturation sensor, blood pressure sensor, heart rate sensor, respiration rate sensor, heart electrical activity sensor, brain activity sensor, or any combination thereof.

According to some embodiments, there is provided a breath monitoring system which include a patient interface unit, adapted to be positioned on the patient head, said patient interface unit including: i. a breathing guide, adapted to direct exhaled breath from a nostril and/or mouth of the patient towards a sampling area in or in proximity to said breathing guide; ii. one or more CO₂ sensors located in the sampling area; iii. a control logic adapted to compute at least one CO₂ related parameter in the exhaled breath; and iv. a transmitter adapted to wirelessly transmit data corresponding to the CO₂ related parameters to a remote unit; and; a remote unit adapted to wirelessly receive from the patient interface unit the data corresponding to the CO₂ related parameters and to further process and/or display said data or information related to said data. The one or more CO₂ sensors of the system may include an infra red based CO₂ sensor, semi-conductor based CO₂ sensor, nanotechnology based CO₂ sensor, or any combination thereof.

According to further embodiments, the patient interface unit of the system may further include one or more power sources, one or more data converters adapted to convert data obtained from the CO₂ sensor into digital data, or a combination thereof.

According to further embodiments the system may further include a carrying unit adapted to position the patient interface on the patient head.

According to yet further embodiments, the at least one CO₂ related parameter include: CO₂ concentration, CO₂ waveform, change in CO₂ concentration over time, End Tidal CO₂ (Et CO₂) or any combination thereof. According to yet further embodiments, the patient interface unit of the system may further include one or more additional sensors. The one or more additional sensors comprise: temperature sensor, blood oxygen saturation sensor, blood pressure sensor, heart rate sensor, respiration rate sensor, heart electrical activity sensor, brain activity sensor, or any combination thereof.

According to some embodiments, there is provided a breath monitoring system which includes: a patient interface unit, adapted to be positioned on the patient head, said patient interface unit comprises a cannula, adapted to collect a sample of exhaled breath from a patient; and a platform unit including: i. one or more CO₂ sensors adapted to sense CO₂ levels in the sample; ii. a pump adapted to transfer the sample from the cannula to the one or more CO₂ sensors; iii. a communication unit adapted to wirelessly transmit data corresponding to the CO₂ levels to a remote analyzing unit; and; a remote analyzing unit adapted to wirelessly receive from the platform unit the data corresponding to the CO₂ levels and to further process and/or display said data or information related to said data.

According to additional embodiments, the platform unit of the system may further include a control center, one or more power sources, one or more adapters, one or more additional sensors, or any combination thereof. The one or more additional sensors may include temperature sensor, blood oxygen saturation sensor, blood pressure sensor, heart rate sensor, respiration rate sensor, heart electrical activity sensor, brain activity sensor, or any combination thereof. The adapters may include adapters for connection to a monitoring device, sampler, O₂ supply, power source, or any combination thereof. According to yet additional embodiments, the platform unit of the system may be adapted to regulate the O₂ supply. Regulation of the O₂ supply may be such that the O₂ supply is stopped or reduced during the patient's exhalation.

According to yet further embodiments, the platform unit of the system may be portable. The platform unit is adapted to be connected with the patient's bed or chair.

According to yet further embodiments, the communication unit of the system may be further adapted to wirelessly receive information and/or data from the analyzing unit. The data corresponding to the CO₂ levels may include CO₂ concentration, CO₂ waveform, change in CO₂ concentration over time, End Tidal CO₂ (Et CO₂) or any combination thereof.

According to yet additional embodiments, the one or more CO₂ sensors comprise an infra red based CO₂ sensor, semi-conductor based CO₂ sensor, nanotechnology based CO₂ sensor, or any combination thereof.

According to further embodiments, the remote main analyzing unit may include a processor subunit, a user interface, display, communication subunit, or any combination thereof.

In addition to the exemplary aspects and embodiments described above, further aspects and embodiments will become apparent by reference to the figures and by study of the following detailed descriptions.

### BRIEF DESCRIPTION OF THE FIGURES

Fig. 1A - A block diagram of a wireless monitoring system, according to some embodiments;
Fig. 1B - Schematic illustration of a perspective view of a monitoring system;
Fig. 1C - Schematic illustration of a perspective view of a wireless monitoring system, according to some embodiments;
Fig. 2A-C - Schematic illustration of a perspective view (Fig. 2A), front view (Fig. 2B) and side view (Fig. 2C) of a patient's head while wearing a sampling unit of a wireless capnography device, according to some embodiments;
Fig. 3 - Schematic illustration of a perspective view (Fig. 3A), front view (Fig. 3B) and side view (Fig. 3C) of a patient's head while wearing a sampling unit of a wireless capnography device, according to some embodiments; and
Fig. 4 - Schematic illustration of a perspective view (Fig. 4A), front view (Fig. 4B) and side view (Fig. 4C) of a patient's head while wearing a sampling unit of a wireless capnography device, according to some embodiments.

### DETAILED DESCRIPTION

In the following description, various aspects of the disclosure will be described. For the purpose of explanation, specific configurations and details are set forth in order to provide a thorough understanding of the disclosure. However, it will also be apparent to one skilled in the art that the disclosure may be practiced without specific details being presented herein. Furthermore, well-known features may be omitted or simplified in order not to obscure the disclosure.

As referred to herein, the term "patient" relates to a subject, which is being monitored by a medical monitoring system. For example, the medical monitoring system may include capnograph that may be used to measure CO₂ levels in exhaled breath of the patient.

As referred to herein, the terms "tube" and "tubing" may interchangeably be used and relate to a physical connection means that is used for the transfer of fluids from a patient to a monitoring system, and/or between various units of the monitoring system.

As referred to herein, the term "wire", "wiring" may be interchangeably used and relate to a physical connection means (such as wire) that may be used to transfer signals other than fluids from a patient to a medical monitoring system, and/or between various units of the medical monitoring system. The signals thus transferred may include any type of signals, data, and power, such as, for example, digital data, electric signals, electrical power, and the like. The connection between the patient and the wires (patient interface) may be direct or indirect and may involve the use of various adaptors and mediators.

As referred to herein, the terms "wireless" and/or "tubeless" relates to reducing or eliminating at least some of the physical connections between various functional units of a medical monitoring system, wherein the monitoring system has more than one separable functional unit. As a result of the reduction/elimination of the physical connection between at least some of the various units of the monitoring system, the system is referred to herein as a wireless and/or tubeless monitoring system. The wireless and/or tubeless monitoring system may include a completely wireless and/or tubeless system wherein all of the separable functional units of the monitoring system are not physically connected. The wireless and/or tubeless monitoring system may include a partially wireless and/or tubeless system, wherein only some of the units of the monitoring system are not physically connected.

As referred to herein, the terms "health care provider", "caregivers", "medical staff' may interchangeably be used and may relate to a health care professional or non-professional that may monitor, track the patient status and/or treat the patient. For example, health care providers may include nurse, physician, therapist and the like.

As mentioned above, in order to address various disadvantages of the use of wires and/or tubes in the connection of a patient and medical monitoring systems and between the various functional units of the monitoring system, several solutions may be used when creating a wireless and/or tubeless medical monitoring system, either completely wireless and/or tubeless or partially wireless and/or tubeless monitoring systems.

According to some embodiments, the wireless medical monitoring system may include a monitoring system that has more than one functional separable unit, and wherein the connection between at least two of the units is wireless and/or tubeless. For example, the medical monitoring system may include an SpO₂ monitoring system, an ECG monitoring system, blood pressure monitoring system, capnography monitoring system, temperature monitoring system, EEG monitoring system, heart rate monitoring system, breath rate monitoring system, and the like, and any combination thereof.

According to some examples, the wireless monitoring system may be divided into three functional units: a patient interface, which includes the unit that interfaces with the patient and is generally used as the unit that is adapted to collect/sense/measure samples or any other data (such as, for example, breath samples) related to the patient. For example, the patient interface may include a sampler, a sensor, a collection element, and the like. For example, the patient interface unit may be used to collect/sense/measure such parameters as: breath samples, CO₂ levels in exhaled breath, blood pressure, blood oxygen saturation, heart rate, heart electrical activity, respiratory rate, and the like, or any combination thereof. Depending on the type of the patient interface unit and the monitoring system, the patient interface unit may further include, for example: a power source that may be used, for example, to operate a sensor or a sensor related element (such as, for example, a pump used for blood pressure measurements); a receiver/transmitter interface adapted to wirelessly communicate with other functional units of the monitoring system, and the like. An additional unit of the monitoring system may include a **platform unit** that may include one or more sensors and a control center. Optionally, the platform unit may be a portable unit. The platform unit may be a screen-less platform. The platform unit may be attached/fixed (permanently or transiently) to the patient bed, chair, body, and the like. For example, the platform unit may be attached to the patient bed and may also be carried, at will, by the patient, by a health care provider, and the like. The platform unit may be functionally connected to the patient interface, wherein the connection between the patient interface and the platform unit may be a physical connection or may include a wireless and/or tubeless connection. For example, the platform unit may be wirelessly and/or tubelessly connected to the patient interface. In such a setting an appropriate wireless transmitter-receiver interface is located within the patient interface unit and the platform unit. For example, the platform unit may be physically connected to the patient interface, for example, by wires and/or tubes. The connection between the patient interface and the platform unit may be used to transfer information/data/signals/fluids that are collected/ sensed/measured/sampled by the patient interface (and samplers/sensors therein) to the platform unit. The platform unit may further include adapters for connection to various additional devices, samplers, connectors, power sources, and the like. The platform unit may include an internal power source, such as, for example, rechargeable battery that may provide power for operation when the platform unit is being carried, for example, during transport of the patient. The platform unit may also operate on mains power, while being positioned on the patient bed. During the time that the platform unit is connected to the mains power, the internal rechargeable battery may be recharged. The platform unit may further include a transmitter/receiver unit adapted to send and receive in a wireless manner data/information/signals from additional units of the monitoring system. In addition to the patient interface and the platform unit, the monitoring system may further include a **main analyzing unit** (station) that may include such constituents and subunits as, but not limited to: processing subunit (having appropriate hardware and/or software) adapted to process/analyze information obtained from the platform unit; patient interface subunit, adapted to allow the user (such as a health care provider) to control operating parameters and other parameters that are related to the monitoring system; one or more display subunits (monitor(s)) adapted to visually display various parameters that are related to the operation of the monitoring system and parameters being monitored/measured by the medical device; a communication unit adapted to send/receive data/information/signals from the platform unit. The communication unit may allow wireless communication between the platform unit and the main analyzing unit and may include a receiver and/or transmitter. Such a setting may allow the main analyzing unit to be placed at any location/distance relatively to the patient, at the convenience of the health care provider.

Such a wireless monitoring system may be useful in overcoming many of the disadvantages of non-wireless/non-tubeless monitoring systems. For example, in critical care settings, where physical connection between the patient and monitoring systems limit and interfere with patient movement, the wireless monitoring system may replace the ad-hoc connection between monitors placed in the room where the patient is hospitalized to a fixed connection from the patient to, for example, the back of the patient bed, where the platform unit is located. For example, the use of such wireless monitoring systems may aid the health care provider in reaching the patient from all directions, since no tubes and/or wires reside outside of the bed where the patient is located. For example, transportation of patients to other locations (such as, x-rays, procedures, and the like) is simplified, since no disconnecting is necessary, as the patient interface remains connected to the platform unit, which by itself is attached to the patient bed. For example, mobility of the patient is greatly enhanced with the use of the wireless monitoring system. This may be achieved since the platform unit may be detached from its location on the bed and be carried with the patient. In addition, the connections between the patient interface and the platform unit may easily be removed and upon removing the connections a message may be transferred to the main analyzing unit such that "disconnected" message is displayed on the remote monitor of the main analyzing unit. In such an at least partially wireless and/or tubeless setting, the tubes and wires are directed to one direction, ridding the bed from the web, providing more comfort for the patient in the bed.

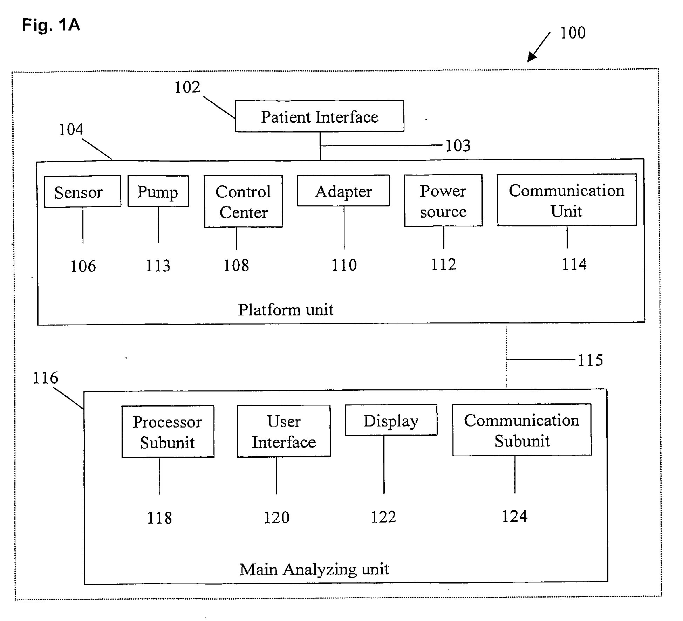

Reference is now made to Fig. 1A, which illustrates a block diagram of a wireless monitoring system 100, according to some embodiments. Patient interface such as patient interface 102, is the interface that is attached or in close proximity to the patient. Patient interface 102 may include one or more samplers, sensors, guides, collectors, and the like that may be adapted to sample/sense/collect a physiological parameter related to the patient. For example, the patient interface may include a breath sampler, such as, for example, a cannula, adapted to sample exhaled breath of the patient. For example, the patient interface may include a heart related sensor (such as, for example, ECG), brain activity related sensor (such as, for example, EEG) a pulmonary related sensor, a blood pressure related sensor (such as, for example, a non-invasive blood pressure (NIBP) cuff), a temperature related sensor (such as, for example, a digital thermometer), a blood related sensor (such as, for example, an SpO₂ sensor), a breath related sensor (such as, for example, a CO₂ sensor), and the like, and any combination thereof. The patient interface may be connected to a platform unit, 104. The connection between the patient interface and the platform unit may be completely or partially wireless and/or tubeless and may involve the use of appropriate transmitter-receiver interface adapted to wireless and/or tubeless connect between the patient interface and the platform unit. Alternatively, or in addition, the connection between the patient interface and the platform unit may include a physical connection, such as by use of wires and/or tubes (as exemplified by connection 103 in Fig. 1A). The connection between the patient interface and the platform unit may be used for the transfer of information/data and/or physical samples (such as for example, fluids of exhaled breath) between the patient interface and the platform unit. The platform unit may be placed in close proximity to the patient, such as, for example, at the patient bed. The platform unit may include several constituents and any combination of constituents, such as, but not limited to: one or more sensors, such as sensor 106, adapted to sense desired parameters in the samples acquired by the patient interface. For example, sensor 106 may include a CO₂ sensor, adapted to measure the CO₂ levels in exhaled breath sample obtained by the patient interface; control center, such as control center 108, adapted to control and coordinate operation of the various constituents of the platform unit and of the patient interface. The control center may be user accessible and may operate automatically; adapters, such as adapter 110, used to allow for connection to various additional devices, samplers, connectors, power sources, and the like. For example, adapter 110 may include one or more connectors to connect to, for example, Oxygen (O₂) supply of the patient. For example, adapter 110 may include connection to a power supply that may provide the platform unit with operating power and may also be used to recharge a rechargeable internal power source, 112, that may reside in the platform unit. In addition, the platform unit may optionally include a pump, such as pump 113. The pump may be used to transfer fluids from, for example, the patient interface to a sensor in the platform unit. For example, a pump may be used with an NIBP cuff, a CO₂ sensor, and the like. In addition, the platform unit may further include a communication unit 114. Communication unit 114 may include receiver and/or transmitter adapted to send and receive information, preferably by wireless routes (shown for example, as wireless route 115 in Fig. 1A), between the platform unit and a remote main analyzing unit (station), 116 and/or to the patient interface, as mentioned above. The main analyzing station 116 may include several subunits, such as, for example, processor subunit 118, adapted to process/analyze/calculate information received from the platform unit. Processor subunit 118 may include any applicable hardware and software. In addition, the main analyzing unit may include a user interface subunit, 120, adapted to allow the user (such as a health care provider) to control operating parameters and other parameters that are related to the monitoring system. The main analyzing unit may further include one or more display subunits (monitor), such as, for example, display 122, adapted to visually display various parameters that are related to the operation of the monitoring system and parameters being monitored/measured by the monitoring system. The main analyzing unit may further include, a communication subunit, 124, adapted to send/receive data/information/signals from the platform unit. Communication subunit 124 may allow wireless communication between communication unit 114 of platform unit 104 and main analyzing unit 116 and may include a receiver and/or transmitter. The wireless communication between platform unit 104 and main analyzing unit 116 may include any type of interface and/or protocol that is known in the art for use in wireless communication. In addition, main analyzing unit 116 may be further adapted to use a transmitter/receiver interface to wirelessly connect to a remote location, such as, for example, a nurse station, a physician's office/clinic, that may be located in a separate room from where the patient and the monitoring system are situated.

According to further embodiments, a platform unit (such as platform unit 104) may be adapted to connect/interact with various main analyzing units. For example, a main analyzing unit may include any type of medical analysis/monitoring system, such as, for example: a capnography main analyzing unit, a blood pressure main analyzing unit, an O₂ saturation (pulse oximetry) main analyzing unit, a heart rate main analyzing unit, a respiratory rate main analyzing unit, an electrocardiography (ECG) main analyzing unit, an electroencephalography (EEG) main analyzing unit, and the like, or any combination thereof.

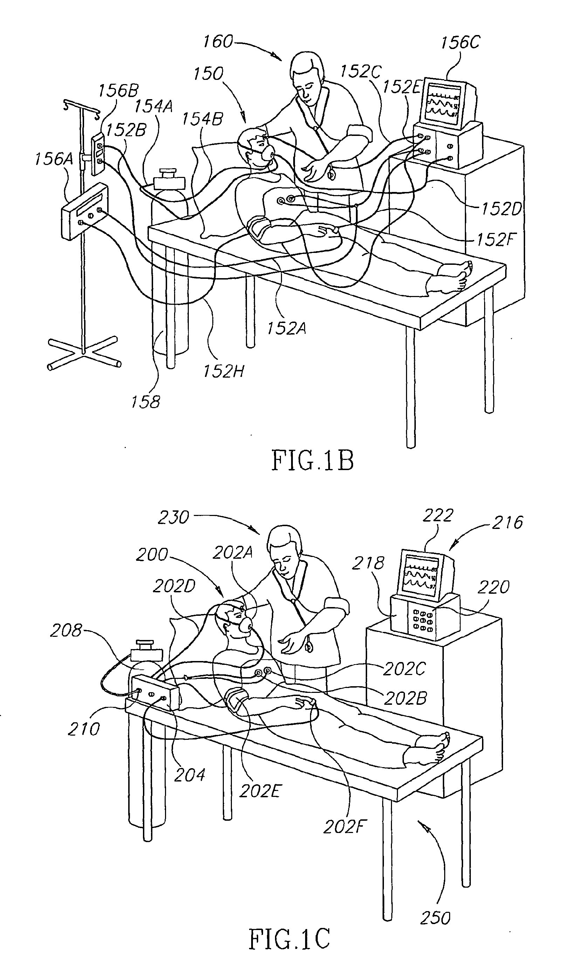

Reference is now made to Fig. 1B, which shows a schematic illustration of a perspective view of a monitoring system, as currently practiced. As shown in Fig. 1B, a patient, such as patient 150, is connected by use of various wires, cables and/or tubes (such as, for example, wires and cables 152A-H; tubes 154A-B) to various monitoring or other health related devices (such as, fore example, monitoring devices 156A-C) and/or various fluid supply (such as, for example, O₂ supply, 158). The various monitoring devices may include such monitoring devices as, but not limited to: a blood pressure monitoring device (such as, for example, NIBP), an O₂ saturation (Oximeter) monitoring device, a heart rate monitoring device, a respiratory rate monitoring device, an electrocardiography (ECG) monitoring device, an electroencephalography (EEG) monitoring device, thermometer, and the like, or any combination thereof. The various fluid supply may include such fluids as, but not limited to: gaseous fluids, such as, for example, Oxygen (02), that may be given via tubes to the patient airway, such as mouth and nose; fluids such as, for example, saline, various drug solutions, such as, for example, antibiotics, analgesics, salts, supplements, and the like, that may be given, for example, via tubes intravenously, into the patient blood circulation. As detailed above and as clearly illustrated in Fig. 1B, the use of the various wires, cables and/or tubes to connect the patient to the various monitoring devices, fluid supply and other health related devices has many disadvantages such as, for example, may prevent an health care provider (such as health care provider, 160), from reaching the patient from all direction, limit patient mobility, and the like.

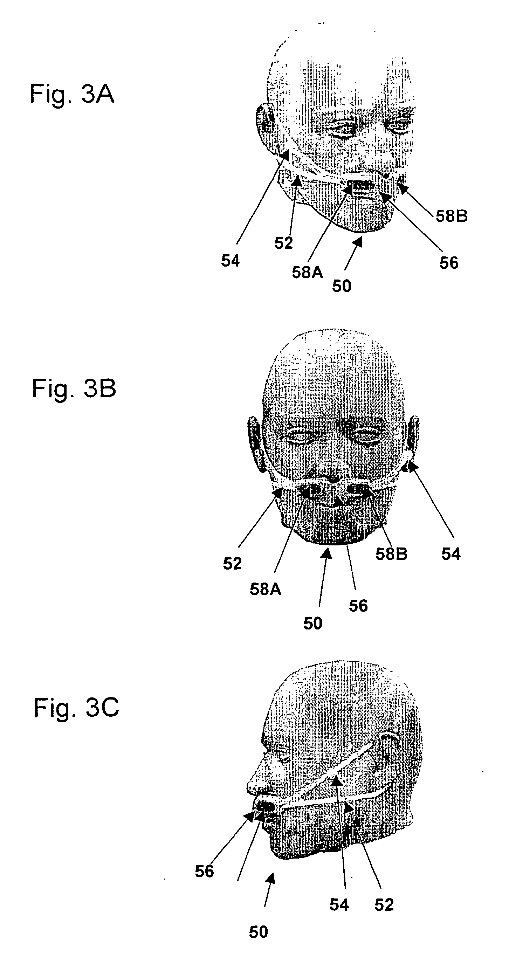

Reference is now made to Fig. 1C, which illustrates a schematic view of a perspective view of a wireless monitoring system, according to some embodiments. As shown in Fig. 1C, to a patient, such as patient 200, a patient interface may be attached or placed in close proximity. The patient interface, may include one or more samplers, sensors, guides, collectors, and the like that may be adapted to sample/sense/collect a physiological parameter related to the patient. As shown in Fig. 1C, the exemplary patient interface includes a combination of sensors/samplers/collectors, marked as patient interfaces 202A-F. For example, the patient interface may include a breath sampler, such as, for example, a cannula, adapted to sample exhaled breath of the patient (such as, for example, patient interface 202A); For example, the patient interface (such as, for example, patient interfaces 202B-C) may include a heart related sensor (such as, for example, ECG).; For example, the patient interface (such as, for example, patient interfaces 202D) may include a brain activity related sensor (such as, for example, EEG); For example, the patient interface (such as, for example, patient interfaces 202E) may include a blood pressure related sensor (such as, for example, a non-invasive blood pressure (NIBP) cuff); For example, the patient interface (such as, for example, patient interfaces 202F) may include a blood related sensor (such as, for example, an SpO₂ sensor); For example, the patient interface may include a pulmonary related sensor (not shown); For example, the patient interface may include a breath related sensor (such as, for example, a CO₂ sensor, (not shown)); For example, the patient interface may include a temperature related sensor (such as, for example, a digital thermometer, (not shown)); and the like, or any combination thereof. The patient interface may be connected to a platform unit, such as, platform unit 204. The connection between the patient interface and the platform unit may be completely or partially wireless and/or tubeless and may involve the use of appropriate transmitter-receiver interface adapted to wireless and/or tubeless connect between the patient interface and the platform unit. Alternatively, or in addition, the connection between the patient interface and the platform unit may include a physical connection, such as by use of wires and/or tubes. The connection between the patient interface and the platform unit may be used for the transfer of information/data and/or physical samples (such as for example, fluids of exhaled breath) between the patient interface (such as, patient interfaces 202A-F) and the platform unit (such as platform unit 204). The platform unit may be placed in close proximity to the patient, such as, for example, at the patient bed (such as bed 250). The platform unit may be portable, such that it may be carried along with the patient. The platform unit may include several constituents and any combination of constituents, such as, but not limited to: one or more sensors, adapted to sense desired parameters in the samples acquired by the patient interface; control center, such as adapted to control and coordinate operation of the various constituents of the platform unit and of the patient interface. The control center may be user accessible and may operate automatically; adapters, used to allow for connection to various additional devices, samplers, connectors, power sources, and the like. For example, adapter 210, is adapted to connect to Oxygen (O₂) supply that may be given to a patient in need. The O₂ supply source may be from a central O₂ supply, or may come from a portable O₂ supply source, such as illustrated for example in Fig. 3C, as a portable O₂ tank, 208). For example, the adapter may include connection to a power supply that may provide the platform unit with operating power and may also be used to recharge a rechargeable internal power source of the platform unit. In addition, the platform unit may optionally include a pump, that may be used to transfer fluids from, for example, the patient interface to a sensor in the platform unit. For example, a pump may be used with an NIBP cuff, a CO₂ sensor, and the like. In addition, the platform unit may further include a communication unit. The communication unit may include receiver and/or transmitter adapted to send and receive information, preferably by wireless routes, between the platform unit and a remote main analyzing unit (station), 216 and/or to the patient interface, as mentioned above. The main analyzing station, such as, main analyzing unit 216 may include several subunits, such as, for example, processor subunit (control logic), adapted to process/analyze/calculate information received from the platform unit. The processor subunit (such as processor subunit 218) may include any applicable hardware and software. In addition, the main analyzing unit may include a user interface subunit, 220, adapted to allow the user (such as a health care provider 230) to control operating parameters and other parameters that are related to the monitoring system. The main analyzing unit may further include one or more display subunits (monitor), such as, for example, display 222, adapted to visually display various parameters that are related to the operation of the monitoring system and parameters being monitored/measured by the monitoring system. The main analyzing unit may further include a communication subunit, adapted to send/receive data/information/signals from the platform unit. The communication subunit may allow wireless communication between the communication unit of platform unit (204) and main analyzing unit 216 and may include a receiver and/or transmitter. The wireless communication between platform unit (204) and main analyzing unit (216) may include any type of interface and/or protocol that is known in the art for use in wireless communication. In addition, main analyzing unit 216 may be further adapted to use a transmitter/receiver interface to wirelessly connect to a remote location, such as, for example, a nurse station, a physician's office/clinic, that may be located in a separate room from where the patient and the monitoring system are situated.

According to some embodiments, and as demonstrated by in Fig. 1A-C, the use of a wireless and/or tubeless monitoring system (such as system 100 in Fig. 1A), may facilitate operation of the monitoring systems by the health care provider, increase accessibility of the medical stuff to the patient and increase comfort and mobility of the patient. In addition, since various sensors/samplers/devices are interfacing the patient through a common unit (the platform unit), which commonly controls the various sensors/samplers/devices, the accuracy of the measurements thus obtained may be greatly enhanced. For example, with respect to capnography monitoring systems, as further detailed below, it is known that supply of O₂ to the patient may interfere with the accuracy of the CO₂ measurements in exhaled breath of the patient, since O₂ may mix and dilute said exhaled breath sample. Therefore, when using a wireless capnography system as described herein, an O₂ cannula, which supplies O₂ to the patient may connect to the platform unit. Likewise, the O₂ supply (such as from the main hospital supply, through a wall connection, or from a portable O₂ cylinder) may also connect to the platform. By using such connections, the user (such as the health care provider) may decide to connect the platform unit to the main hospital supply, for example when the patient is in his room, or to connect the platform unit to the portable O₂ cylinder, for example, when the patient is transferred with his bed to X-ray, operation room or any other place. Furthermore, since the O₂ is delivered through the platform unit, which also include the controls and CO₂ sampling elements, a temporal coordination between supplying O₂ and measuring CO₂ may be achieved. For example, to this aim, O₂ may only be delivered during inhalation, thus reducing dilution of the CO₂ in the exhaled breath, which is a common limitation when using capnography with non-intubated patients. Alternatively, or in addition, during exhalation O₂ flow levels may be reduced such that the dilution effect is minimal. For example, O₂ flow levels may be at 3-4 L/min during inhalation and reduced to 1L/min during exhalation. Reducing or stopping O₂ flow during exhalation may also save O₂ and thus cost. In addition, Reducing or stopping O₂ flow during exhalation may lower the O₂ concentration in the patient's near environment and thus reduce the chance of ignition of the highly inflammable O₂ by a spark, which may be caused, for example, by a cigarette or by an electrical device.

According to some embodiments, there is provided a controller adapted to regulate O₂ supply, such that the O₂ supply will be stopped or reduced during exhalation. The controller may be a part of a platform unit or a part of any other device or system which may or may not be functionally connected with a platform unit. The controller may be adapted to regulate O₂ supply based on a signal indicative of inhalation and/or exhalation. The signal may be received from any detector and/or sensor such as a capnograph, CO₂ sensor, chest movement detector, flow meter or any other detector and/or sensor.

According to some embodiments, any of the wireless and/or tubeless monitoring system described herein may include any type of interface and/or protocol that is known in the art for use in wireless communication, such as, for example, that described in US Patent 7,215,991 which relates to wireless medical diagnosis and monitoring equipment. .

According to some exemplary embodiments, the wireless and/or tubeless monitoring system may include a completely wireless and/or tubeless monitoring system. As an example, a completely wireless and/or tubeless capnography that may address at least some of the disadvantages of using wires/tubes with monitoring systems may be used. Capnography is a non-invasive monitoring method used to continuously measure CO₂ concentration in exhaled breath. Based upon the location of the CO₂ sensor, capnographers may be divided into two groups: mainstream and sidestream. In mainstream capnography, a CO₂ sensor is located directly between the airway tube and the breathing circuit, and as such, mainstream capnography is primarily limited to use on intubated patients. In sidestream capnography, the CO₂ sensor is remote from the patient and it is located in the main unit. A sample of the exhaled breath is aspirated by pumps from the airway towards the CO₂ sensor in the main unit. This method can be used with both intubated patients, by connecting to the intubation tube and with non-intubated patients, by connecting to a mask or nostrils of the patient. By this method, capnography can be simultaneously performed with other airways-involving procedures such as oxygen administration.

In general, mainstream capnography is tubeless, and side-stream capnography is wireless. Wireless mainstream capnography may only be used with intubated patients, since it is dependant on the fact that there is a patient airway tubing. For the non-intubated patient, mainstream capngoraphers have nothing to latch/attach to on the patient interface and therefore may not be as useful, unless "bagging" of the patient (manual ventilation) is performed. On the other hand, sidestream capnography is wireless, but needs a pump or the like to suck/take in a sample of the patients breath towards the remote main unit for detection, monitoring, analysis, and the like, of the CO₂ levels. Usually, sidestream capnography sampling systems and patient interfaces are designed while taking into consideration that a pump creating negative pressure is being used. In a completely wireless, tubeless capnograph, the design of the sampling unit must thus be changed so that this crucial difference is addressed.

According to some embodiments, there are several design considerations and constraints that are taken into consideration when designing and creating patient sampling interfaces to be used with completely wireless and/or tubeless capnography, whether a pump is used or not. Such considerations may include, for example: 1. the natural physiology of the mammalian breathing system is designed so that during exhalation stage, the exhaled breath, which is rich in CO₂, may be efficiently dispersed well into the atmosphere so that the immediately proceeding inhalation stage will consist of fresh atmospheric air with minimal CO₂ content. Hence, after leaving the body's breathing cavities (such as nose and mouth), there is no major region/area, in which the exhaled breath collects and can be used as a preferred site for measuring CO₂ content. This dispersing mechanism of the exhaled breath may be created by several mechanisms, such as, for example: the positive pressure and high breath flow rate that is created while exhaling (for example, by the thoracic muscle, diaphragm and the like) via small diameter nostrils; the exhaled breath is directed downwards by the nose and outward by mouth; the higher temperature of the exhaled breath as compared to the atmospheric air, creates a general upward flow after being directed downwards from the mouth. 2. a patient may alternate his breathing pattern, such as, for example, mainly breathing through his nose, but may also breath through his mouth (typical, for example with deeply sedated patients, congested patients, Obstructive sleep apnea patients, and the like). Hence the wireless sampling system (interface) may be adapted to sample from the nose and/or the mouth. 3. In general practice, non-intubated patients often receive supplementary O₂. The O₂ is provided at flow rates of the same order of magnitude as the patient breathing parameters (for example, in the range of 2 to 10 liters/minute). These high flow rates of O₂ may dilute the breath to be sampled. 4. The patient's nostrils and/or mouth airways may not be sealed off in order to trap and monitor the patient's breath. In addition, no interfering or restricting the patient from breathing freely is acceptable. 5. The use of a pump becomes extremely important when relating to the CO₂ response times, where a crisp waveform requires fast responses to change. The pump is important since it is constantly cleaning the sampling region from the inhalation and exhalation stages that pass by it. In addition, the pump may permit the use of small diameter tubing, which otherwise may be restricting the free flow, and thus effecting the response time. Hence in a design with a miniature pump integrated, a cannula design using a "Y" junction and holes for O₂ delivery may be used. Such a cannula design has been detailed, for example, in US Patent 6,422,240. 6. In order to provide an advantage of mobility, wireless transmission/reception protocols may be used to determine which wireless interface transmits to which monitoring system and which monitoring system displays the information of which patient. Such communication protocols may also be used to prevent different interfaces from communicating with the same monitoring system and vice versa. Such circumstances may arise, for example, when several patients are being monitored by wireless. 7. Zeroing process of the CO₂ detectors may be performed by using the ambient atmospheric air as a "zero" reading (which is sensitive to high CO₂ levels in the ambient air, especially close to the patients nose).

The constraints and consideration for a completely wireless and/or tubeless capnography, such as those presented above herein may dictate novel designs to permit accurate sampling of the exhaled CO₂ as well as ease of use. These novel designs together with the negative pressure created by the miniature pump may reduce the chance that the monitoring system will provide erroneous results.

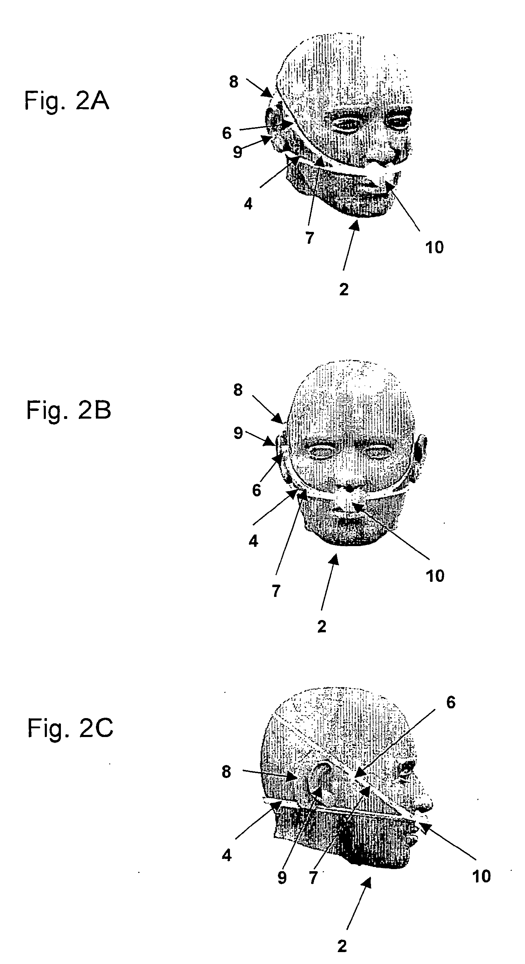

According to some embodiments, there is provided a completely wireless/tubeless capnograph with a miniature mechanical pump for sampling breath samples of a patient. Such wireless capnograph may be designed, which has no tubes that are connected to the remote main unit. Reference is now made to Fig. 2, which schematically illustrates a perspective view (Fig. 2A), Front view (Fig. 2B) and side view (Fig. 2C) of a patient's head while wearing a patient interface unit of a wireless capnography device, according to some embodiments. As shown in any of Figures 2A-2C, carrying unit (shown as carrying straps, 4) are used to position the various constituents of the patient interface unit, onto its location on the patient face (2). The patient interface unit may include various constituents, such as, for example, cannula 10, which may be used to direct exhaled breath from the nostrils and/or mouth of a patient to a desirable location, (such as, for example, a sampling area); a CO₂ sensor (6) that may be used to detect/determine/analyze CO₂ levels in at least a sample of the exhaled breath; and a housing (such as housing 8). In housing 8, which may be located behind the patient's ear (illustrated in Fig. 2, behind right ear, 9), various other constituents of the patient interface unit may be positioned, such as, for example, a miniature low powered pump used to pump a breath sample from cannula 10 (for example, from the sampling area) towards the CO₂ sensor (6). The pump may be used to pump at low flow rates (and thus allow low power consumption), such as, for example at a flow rate of 20ml/min and with minimal resistance since the tubing (7) over which length the fluids are pumped may be short, such as the distance from the mouth to the location of the pump (in this example, the patient ears). In addition, housing 8 may include one or more power sources, such as, for example, a rechargeable battery that may provide the necessary power needed to operate the miniature mechanical pump; a control logic adapted to compute CO₂ related parameters in the exhaled breath, one or more data converters used to convert data obtained from the CO₂ sensor into digital data; one or more transmitters and/or antennas, adapted to transmit the data to the remote main unit; or any combination thereof.

According to other embodiments, there is provided a wireless and/or tubeless capnograph wherein no pump is used with the patient interface unit. Such wireless capnograph may require the use of a measuring sensor that may use one or more sensing technologies to monitor CO₂ levels. For example, one such technology may include the Infra-Red (IR) technology method. For example, another such technology may include the semi-conductor technology method. For example, another such technology may include nanotechnology.

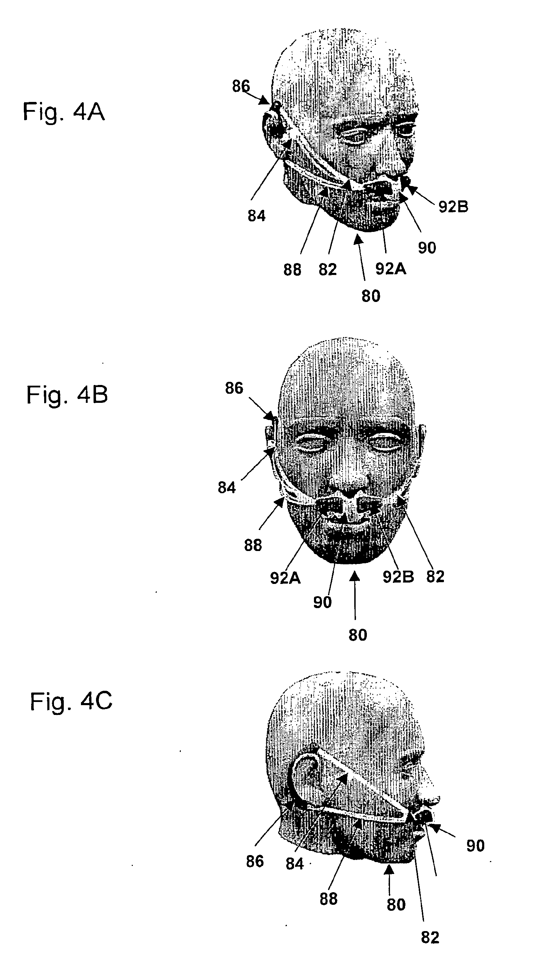

The IR technology method utilizes the spectral characteristics of the CO₂ molecule, which absorbs (and emits) radiation in the IR region of about 4.3 microns. Common elements of this technology is an IR source of radiation, an optical detector and a space (channel) between them, over which gas sample is passed through. Sometimes it may be difficult to optimally position the sensor in proximity to the nose and mouth. This may be solved by using optical fibers at, for example, 4.3 microns, while retaining the source, detector (sensor) and power source at a location, which is remote from the mouth (such as for example, seated on the ear or a similar position that would not interfere, attached to a strap situated on the patients face, and the like, such as illustrated, for example, in Fig. 4, herein).

The semi-conductor technology involves the use of sensors that consist of semi-conductors that may include a sensitive coating layer (such as those known in the art as nano-chains). When the sensitive layer is attracted (adsorbs) to molecules such as CO₂, their electric characteristics are changed. Thus, the electric characteristics are proportional to the concentration of the molecule and can be measured. This type of detectors may be very small in size and may require low power to operate and hence may be appropriate for use with a wireless capnography device, such as illustrated, for example, in Fig. 3 herein.

According to some embodiments, there are several design considerations and constraints that are taken into consideration when designing and creating patient sampling interfaces to be used with completely wireless and/or tubeless capnography that does not involve the use of miniature pumps. Such considerations may include, for example: 1. Necessity to provide free, unrestricted air flow. 2. Sampling should be performed at regions located before dispersion of exhaled CO₂ into ambient air, in order to avoid dilution of the exhaled CO₂ in ambient air. 3. Sampling of the CO₂ should be separated from O₂ flow, if O₂ supply is provided to the patient to reduce the chance of dilution.

Reference is now made to Fig. 3, which schematically illustrates a perspective view (Fig. 3A), Front view (Fig. 3B) and side view (Fig. 3C) of a patient's head while wearing a patient interface unit of a completely wireless capnography device, according to some embodiments. As shown in any of Figures 3A-C, carrying unit (shown as carrying straps, 52) are used to position the various constituents of the patient interface unit, onto its location on the patient face (50). The patient interface unit may include various constituents, such as cannula 56, which is used to direct breath from the nostrils and/or mouth of a patient towards the sampling area wherein housing 58A and 58B are located. In housings 58A and 58B, which may be located to the sides of cannula 56, various other constituents of the patient interface unit may be located, such as, for example, CO₂ sensor; one or more power sources, such as rechargeable battery; a control logic adapted to compute CO₂ related parameters in the exhaled breath; one or more data converters used to convert data obtained from the CO₂ sensor into digital data; one or more transmitters, adapted to transmit the data to the remote main unit; or any combination thereof. In addition, antenna, such as antenna 56 may be identified that may aid in transmitting data from the transmitter towards the remotely located main unit.

Reference is now made to Fig. 4, which schematically illustrates a perspective view (Fig. 4A), Front view (Fig. 4B) and side view (Fig. 4C) of a patient's head while wearing a patient interface unit of a wireless capnography device, according to some embodiments. As shown in any of Figures 4A-C, carrying unit (shown as carrying straps, 82) is used to position the various constituents of the patient interface unit, onto its location on the patient's face (80). The patient interface unit may include various constituents, such as breathing guide 90, which is used to direct breath from the nostrils and/or mouth of a patient towards the sampling area wherein one or more CO₂ sensors (shown as two sensors, 92A and 92B) are located. As shown in Figs. 4A-C, the one or more CO₂ sensors may be located in close proximity to the breathing guide. The CO₂ sensors may include any type of sensor known in the art, as detailed below herein, and may be used to directly or indirectly sense/measure CO₂ related parameters of at least a sample of the patient's breath. The information received by the CO₂ sensors may be transferred to housing 86, which may be located behind the patient ear, and may include various other constituents of the patient interface unit, such as, for example one or more power sources, such as rechargeable battery; a control logic adapted to compute CO₂ related parameters in the exhaled breath ; one or more data converters used to convert data obtained from the CO₂ sensor into digital data; one or more transmitters, adapted to transmit the data to the remote main unit; or any combination thereof. The data transferred from the CO₂ sensors (92A and 92B) towards housing 86, which includes the additional constituents of the patient interface unit, may be transferred, for example, by the use of optical fibers, such as, for example, optical fibers 88. In addition, antenna, such as antenna 84 may be identified that may aid in transmitting data from the transmitter towards the remotely located main unit. In addition, or alternatively, data may be transferred by other means, such as, for example, electrical wires.

Various examples of specific settings, considerations and configurations of patient interface units of wireless capnograph device, which has been schematically illustrated in Figures 2-4 are described herein.

According to additional embodiments, one nostril of the patient may be used for O₂ delivery. All (100%) or at least the majority (> 75%) of O₂ may be delivered to one nostril while the remaining dosage of O₂ may be delivered either to the mouth or to the second nostril through a tube with small holes that are spread out. The second nostril may be used for sampling of exhaled CO₂. The holes for O₂ delivery on the nostril side where the sampling of CO₂ is also performed may be covered with light and thin plastic flap, so that during inhalation the force of the O₂ delivery opens the flap easily, but during exhalation the force from the exhaled breath is sufficient to close off the flap or at least to divert the direction of the O₂. Such a setting may also be used for reducing the dilution effect even when 50% of O₂ is provided to each nostril.

According to additional embodiments, the patient interface unit may include a CO₂ sampling unit and may further include a nasal interface that may include a prong that may be used to direct the nasal breath towards the sensing/measuring region of the sampling unit. The diameter of the CO₂ sampling nasal prong may be large, such as, for example at least 1.6 mm and higher. The diameter of the CO₂ sampling nasal prong may be about 5mm. Further more, the diameter of the CO₂ sampling nasal prong should not reduce in size as it exits the nostril.

According to further embodiments, when a nasal prong type is used, it may also be combined with an oral scoop, such as described, for example in US Patent 6,422,240. In such a setting, the "Y" Junction of the prong may be the location where the CO₂ sensor is placed. For example, when using an IR radiation/detector CO₂ sensor, this may be the measuring location (such as illustrated in Fig. 3A-C and Fig. 4A-C). Likewise, when using a semi-conductor type sensor, this may be the location on which the semiconductor-type sensor is positioned (such as illustrated in Fig. 3A-C and Fig. 4A-C).

According to other embodiments, a nasal prong may not necessarily be used and inserted into the nostril. Rather, a breathing guide, that may include various types of soft cover may be placed over the nose or one or two nostrils of the patient, such that the exhaled breath is not dispersed in all directions when leaving the nostril but may be guided as an extension of the nose to a desired region/location where the CO₂ levels are more easily measure. For example, the CO₂ sensor may be located in that region. In addition, optical fibers may be used to connect between the breathing guide and the CO₂ sensor.

According to further embodiments, the breathing guide may further be placed such that it may extend towards the patients mouth so that also mouth breathing may be guided towards the CO₂ measuring sensor. The guiding section for the oral breath may be connected to the bottom part of the patients lip since when a patient opens his mouth the bottom lip is moved. Since the patient breath is warmer than the ambient atmospheric air, the exhaled air tends to disperse upwards, hence guiding the exhaled upwards with the aid of the breathing guide actually helps the exhaled air to move in its natural direction.

According to further embodiments, a fixed distance between the source of radiation (such as, for example, IR emission) and the detector of the CO₂ sensor may be defined by the breathing guide. Likewise, a distance may be defmed between the two ends of an optical fiber that are positioned in the breath flow pathway that is determined by the breathing guide (as exemplified in Fig. 4A-C). In such a setting, an enclosed absorption cell into which exhaled breath may be collected is not necessary, since the breathing guide is sufficient to create a defined pathway over which at least a sample of the exhaled CO₂ may be measured.

According to some embodiments, the source of radiation (such as IR) and detector of the CO₂ sensor may not need be on different sides of the exhaled air flow pathway. The breath guide may have a reflective surface that may allow the radiation source and detector to be found on the same side. In such a setting, the radiation from the radiation source may be reflected from the reflective surface of the breathing guide towards the CO₂ detector.

According to other embodiments, some constituents of the patient interface unit, such as for example, cannulas, nostril cannulas, breathing guide, and the like, may be of the disposable type. Other constituents, such as housing, CO₂ sensor, power source, transmitters, data converters and additional attached subunits may be reusable with an appropriate interface.

According to some embodiments, there is provided a vital-sign monitoring system with minimal physical connectivity between the patient and the monitoring system. Preferably, the connectivity between the patient and the monitoring system is completely wireless and tubeless. In such a system, the carrying (positioning) unit (such as an headset) of the wireless capnograph units (such as, for example, the patient interface unit, which may include a CO₂ sensor, exhaled breath sampling interface, miniature mechanical pump, power source, transmitter, data converter, and the like or any combination thereof) may also be attached to additional medical sensor/sampling unit. All monitoring/sampling units may be connected to one carrying unit (such as, for example, a headset), with a common communication unit (that may include, for example, a transmitter) and a common power supply. The additional medical sensor/sampling unit may include any type of medical sensor that may be used to detect/sense/measure health related physiological condition of a patient. For example, but not limited to, the additional medical sensor/sampling unit may include breath related sensor, such as, for example a breath flow meter (for example, of the thermocouple type); a respiratory rate sensor; a temperature sensor (such as a thermometer that may be placed in close proximity to the patient skin); a blood related sensor, such as a blood pressure monitor (for example, that may be attached to a patient ear lobe); an SpO₂ sensor that may be used to measure non-invasively blood oxygenation (O₂ saturation level), most appropriately being of the type that connects to the ear lobe; An electroencephalography (EEG) device that may be used to measure electrical activity of the brain; and the like and any combination thereof.

According to some embodiments, there is provided a monitoring system for combined wireless capnography and oxygen saturation measurements. The wireless capnography may include any of the types of wireless capnography device described herein. Oxygen saturation may be measured, for example, by a pulse oximeter, which is a medical device that may be used to indirectly measure oxygen saturation of a patient's blood. Typically, a pulse oximeter probe has a pair of small light-emitting diodes (LEDs) facing a photodiode through a translucent part of the patient's body. One LED is red, with wavelength of 660 nm, and the other is infrared, (for example at the range of 905, 910, or 940 nm). Absorption at these wavelengths differs significantly between oxygenated blood (which contain oxyhemoglobin) and its deoxygenated form, therefore from the ratio of the absorption of the red and infrared light the oxy/deoxyhemoglobin ratio can be calculated and hence, a measure of oxygenation (the per cent of hemoglobin molecules bound with oxygen molecules) can be made. The pulse oximetry probe may include any type of known pulse oximetry probes, both reusable and disposable such as, for example a finger probe (which attaches to the patient finger), an ear lobe probe (which is attached to the patient ear lobe), a forehead probe (which is attached to a patient forehead), a foot probe (which may be attached to a patient, (usually a neonatal) patient), and any combination thereof. According to some embodiments, data obtained from the wireless capnography sensor and data obtained from

According to further embodiments, there is provided a wireless monitoring system for combined wireless capnograph, wireless pulse oximetry and wireless respiratory rate measurement. The respiratory rate sensor may include any type of wireless respiratory rate sensor, such as, for example, an acoustic respiratory rate sensor. The wireless capnograph may include any of the types described above herein. The pulse oximetry probe may include any type of known pulse oximetry probes, both reusable and disposable such as, for example a finger probe (which attaches to the patient finger), an ear lobe probe (which is attached to the patient ear lobe), a forehead probe (which is attached to a patient forehead), a foot probe (which may be attached to a patient, (usually a neonatal) patient), and any combination thereof.

According to further embodiments, there is provided a wireless monitoring system for combined wireless pulse oximetry and wireless respiratory rate measurement. The respiratory rate sensor may include any type of wireless respiratory rate sensor, such as, for example, an acoustic respiratory rate sensor. The pulse oximetry probe may include any type of known pulse oximetry probes, both reusable and disposable such as, for example a finger probe (which attaches to the patient finger), an ear lobe probe (which is attached to the patient ear lobe), a forehead probe (which is attached to a patient forehead), a foot probe (which may be attached to a patient, (usually a neonatal patient), and any combination thereof.

According to further embodiments, there is provided a wireless monitoring system for combined wireless capnograph and respiratory rate measurement. The respiratory rate sensor may include any type of wireless respiratory rate sensor, such as, for example, an acoustic respiratory rate sensor. The wireless capnograph may include any of the types described above herein.

According to additional embodiments, there is provided a wireless monitoring system for combined wireless capnography and non-invasive blood pressure measurements. The non-invasive blood pressure measurements may be performed by any of the non-invasive methods known in the art, such as, for example, oscillatory methods, oscillometric methods, or any combination thereof. The wireless capnograph may include any of the types described above herein.

According to additional embodiments, there is provided a wireless monitoring system for combined wireless oxygen saturation and non-invasive blood pressure measurements. The non-invasive blood pressure measurements may be performed by any of the non-invasive methods known in the art, such as, for example, oscillatory methods, oscillometric methods, or any combination thereof. The pulse oximetry probe may include any type of known pulse oximetry probes, both reusable and disposable such as, for example a finger probe (which attaches to the patient finger), an ear lobe probe (which is attached to the patient ear lobe), a forehead probe (which is attached to a patient forehead), a foot probe (which may be attached to a patient, (usually a neonatal) patient), and any combination thereof.

According to additional embodiments, there is provided a wireless monitoring system for combined respiratory rate measurements and non-invasive blood pressure measurements. The non-invasive blood pressure measurements may be performed by any of the non-invasive methods known in the art, such as, for example, oscillatory methods, oscillometric methods, or any combination thereof. The respiratory rate sensor may include any type of respiratory rate sensor, such as, for example, an acoustic respiratory rate sensor.

According to further embodiments, there is provided a wireless monitoring system for combined wireless capnograph, wireless pulse oximetry, non-invasive blood pressure measurements and wireless respiratory rate measurement. The respiratory rate sensor may include any type of wireless respiratory rate sensor, such as, for example, an acoustic respiratory rate sensor. The wireless capnograph may include any of the types described above herein. The pulse oximetry probe may include any type of known pulse oximetry probes, both reusable and disposable such as, for example a finger probe (which attaches to the patient finger), an ear lobe probe (which is attached to the patient ear lobe), a forehead probe (which is attached to a patient forehead), a foot probe (which may be attached to a patient, (usually a neonatal) patient), and any combination thereof. The non-invasive blood pressure measurements may be performed by any of the non-invasive methods known in the art, such as, for example, oscillatory methods, oscillometric methods, or any combination thereof.

While a number of exemplary aspects and embodiments have been discussed above, those of skill in the art will recognize certain modifications, permutations, additions and sub-combinations thereof. It is therefore intended that the following appended claims and claims hereafter introduced be interpreted to include all such modifications, permutations, additions and sub-combinations as are within their true spirit and scope.

## Claims

1. A breath monitoring device comprising:
a patient interface unit adapted to be positioned on the patient's head, said patient interface unit comprising a cannula for collecting a sample of exhaled breath from the patient; and
a platform unit , wherein said platform unit comprises:
one or more carbon dioxide (CO₂) sensors for sensing CO₂ levels in the sample,
a pump for transferring the sample from the cannula to the one or more CO₂ sensors,
and
a communication unit configured to wirelessly transmit data corresponding to the CO₂
levels to a remote analyzing unit **characterized in that** said platform unit is configured to be attached to the patient's bed or chair and physically connected to said patient interface unit.

2. The device according to claim 1, wherein the platform unit further comprises a control center, one or more power sources, one or more adapters, one or more additional sensors, or any combination thereof.

3. The device according to claim 2, wherein the one or more additional sensors comprise a temperature sensor, a blood oxygen saturation sensor, a blood pressure sensor, a heart rate sensor, a respiration rate sensor, a heart electrical activity sensor, a brain activity sensor, or any combination thereof.

4. The device according to claim 2, wherein said adapters comprise adapters for connection to a sampler, O₂ supply, a power source, or any combination thereof.

5. The device according to claim 4, wherein said platform unit is configured to regulate the O₂ supply.

6. The device according to claim 5, wherein said platform unit is configured to regulate the O₂ supply based on a signal indicative of inhalation and/or exhalation received from said one or more CO₂ sensors, such that the O₂ supply is stopped or reduced during the patient's exhalation.

7. The device according to claim 1, wherein the platform unit is portable.

8. The device according to claim 1, wherein the communication unit is further configured to wirelessly receive information and/or data from the analyzing unit.

9. The device according to claim 1, wherein the data corresponding to the CO₂ levels comprises concentration, a CO₂ waveform, a change in CO₂ concentration over time, End Tidal CO₂ (Et CO₂) or any combination thereof.

10. The device according to claim 1, wherein the one or more CO₂ sensors comprise an infra red based CO₂ sensor, a semi-conductor based CO₂ sensor, a nanotechnology based CO₂ sensor, or any combination thereof.

11. A breath monitoring system comprising:
a patient interface unit, adapted to be positioned on the patient head, said patient interface unit comprising a cannula for collecting a sample of exhaled breath from the patient;
a platform unit , said platform unit comprising:
one or more CO₂ sensors for sensing CO₂ levels in the sample,
a pump for transferring the sample from the cannula to the one or more CO₂ sensors,
and
a communication unit configured to wirelessly transmit data corresponding to the CO₂
levels; and
a remote analyzing unit configured to wirelessly receive from the platform unit the data corresponding to the CO₂ levels and to further process and/or display said data or information related to said data
**characterized in that** said platform unit is configured to be attached to the patient's bed or chair and physically connected to said patient interface unit .

12. The system according to claim 11, wherein the platform unit further comprises a control center, one or more power sources, one or more adapters, one or more additional sensors, or any combination thereof.

13. The system according to claim 12, wherein the one or more additional sensors comprise a temperature sensor, a blood oxygen saturation sensor, a blood pressure sensor, a heart rate sensor, a respiration rate sensor, a heart electrical activity sensor, a brain activity sensor, or any combination thereof.

14. The system according to claim 13, wherein said adapters comprise adapters for connection to a sampler, O₂ supply, a power source, or any combination thereof.

15. The system according to claim 14, wherein said platform unit is configured to regulate the O₂ supply.

16. The system according to claim 15, wherein said platform unit is configured to regulate the O₂ supply based on a signal indicative of inhalation and/or exhalation received from said one or more CO₂ sensors, such that the O₂ supply is stopped or reduced during the patient's exhalation.

17. The system according to claim 11, wherein the platform unit is portable.

18. The system according to claim 11, wherein the communication unit is further configured to wirelessly receive information and/or data from the analyzing unit.

19. The system according to claim 11, wherein the data corresponding to the CO₂ levels comprises CO₂ concentration, a CO₂ waveform, a change in CO₂ concentration over time, End Tidal CO₂ (EtCO₂) or any combination thereof.

20. The system according to claim 11, wherein the one or more CO₂ sensors comprise an infra red based CO₂ sensor, a semi-conductor based CO₂ sensor, a nanotechnology based CO₂ sensor, or any combination thereof.

21. The system according to claim 11, wherein the remote analyzing unit comprises a processor subunit, a user interface, a display, a communication subunit, or any combination thereof.

## Patentansprüche

1. Atemüberwachungsvorrichtung umfassend:
Eine Patienteninterfaceeinheit, die angepasst ist, an dem Kopf des Patienten angeordnet zu werden, worin die Patienteninterfaceeinheit eine Kanüle zum Sammeln einer Probe aus Ausatemluft des Patienten umfasst, und
eine Plattformeinheit, worin die Plattformeinheit umfasst:
Einen oder mehrere Kohlenstoffdioxid (CO₂) -Sensoren zum Erfassen von CO₂-Spiegeln in der Probe,
eine Pumpe zum Übertragen der Probe aus der Kanüle an den einen oder die mehreren CO₂-Sensoren, und
eine Kommunikationseinheit, die angepasst ist, die den CO₂-Spiegeln
entsprechende Daten an eine entfernte Analyseeinheit kabellos zu übertragen,
**dadurch gekennzeichnet, dass** die Plattformeinheit angepasst ist, mit einem Bett oder Stuhl des Patienten und körperlich mit der Patienteninterfaceeinheit verbunden zu werden.

2. Vorrichtung nach Anspruch 1, worin die Plattformeinheit weiterhin ein Steuerzentrum, eine oder mehrere Stromquellen, einen oder mehrere Adapter, einen oder mehrere zusätzliche Sensoren oder jegliche Kombination davon umfasst.

3. Vorrichtung nach Anspruch 2, worin der eine oder die mehreren zusätzlichen Sensoren einen Temperatursensor, einen Sauerstoffsättigungssensor für Blut, einen Blutdrucksensor, einen Herzfrequenzsensor, einen Atemfrequenzsensor, einen Sensor für die elektrische Aktivität des Herzens, einen Hirnaktivitätssensor oder jegliche Kombination davon umfasst.

4. Vorrichtung nach Anspruch 2, worin die Adapter Adapter für eine Verbindung zu einem Probennehmer, einer O₂-Quelle, einer Stromquelle oder jegliche Kombination davon umfassen.

5. Vorrichtung nach Anspruch 4, worin die Plattformeinheit angepasst ist, die O₂-Versorgung zu steuern.

6. Vorrichtung nach Anspruch 5, worin die Plattformeinheit angepasst ist, die O₂-Versorgung auf Grundlage eines Signals zu steuern, das eine Ausatmung und/oder Einatmung anzeigt und von dem einen oder den mehreren CO₂-Sensoren erhalten wird, so dass die O₂-Versorgung gestoppt oder reduziert wird, während der Patient ausatmet.

7. Vorrichtung nach Anspruch 1, worin die Plattformeinheit transportabel ist.

8. Vorrichtung nach Anspruch 1, worin die Kommunikationseinheit weiterhin angepasst ist, Informationen und/oder Daten von der Analyseeinheit kabellos zu empfangen.

9. Vorrichtung nach Anspruch 1, worin die Daten, die den CO₂-Spiegeln entsprechen, eine Konzentration, eine CO₂-Wellenfarm, eine Änderung in der CO₂-Konzentration über die Zeit, endexspiratorisches CO₂ (EtCO₂) oder jegliche Kombination davon umfassen.

10. Vorrichtung nach Anspruch 1, worin der eine oder die mehreren CO₂-Sensoren einen auf Infrarot basierenden CO₂-Sensor, einen auf Halbleiter basierenden CO₂-Sensor, einen auf Nanotechnologie basierenden CO₂-Sensor oder jegliche Kombination davon umfassen.

11. Atemüberwachungssystem umfassend:
Eine Patienteninterfaceeinheit, die daran angepasst ist, an dem Kopf des Patienten angeordnet zu werden, worin die Patienteninterfaceeinheit eine Kanüle zum Sammeln einer Probe aus Ausatemluft des Patienten umfasst, und
eine Plattformeinheit, worin die Plattformeinheit umfasst:
Einen oder mehrere Kohlenstoffdioxid (CO₂) -Sensoren zum Erfassen von CO₂-Spiegeln in der Probe,
eine Pumpe zum Übertragen der Probe aus der Kanüle an den einen oder die mehreren CO₂-Sensoren, und
eine Kommunikationseinheit, die angepasst ist, den CO₂-Spiegeln entsprechende Daten kabellos zu übertragen, und
eine entfernte Analyseeinheit, die angepasst ist, die den CO₂-Spiegeln entsprechende Daten kabellos von der Plattformeinheit zu erhalten und diese Daten oder die auf diese
Daten bezogenen Informationen weiter zu verarbeiten und/oder anzuzeigen,
**dadurch gekennzeichnet, dass** die Plattformeinheit angepasst ist, mit einem Bett oder Stuhl des Patienten und körperlich mit der Patienteninterfaceeinheit verbunden zu werden.

12. System nach Anspruch 11, worin die Plattformeinheit weiterhin ein Steuerzentrum, eine oder mehrere Stromquellen, einen oder mehrere Adapter, einen oder mehrere zusätzliche Sensoren oder jegliche Kombination davon umfasst.

13. System nach Anspruch 12, worin der eine oder die mehreren zusätzlichen Sensoren einen Temperatursensor, einen Sauerstoffsättigungssensor für Blut, einen Blutdrucksensor, einen Herzfrequenzsensor, einen Atemfrequenzsensor, einen Sensor für die elektrische Aktivität des Herzens, einen Hirnaktivitätssensor oder jegliche Kombination davon umfasst.

14. System nach Anspruch 13, worin die Adapter Adapter für eine Verbindung zu einem Probennehmer, einer O₂-Quelle, einer Stromquelle oder jegliche Kombination davon umfassen.

15. System nach Anspruch 14, worin die Plattformeinheit angepasst ist, die O₂-Versorgung zu steuern.

16. System nach Anspruch 15, worin die Plattformeinheit angepasst ist, die O₂- Versorgung auf Grundlage eines Signals zu steuern, das eine Ausatmung und/oder Einatmung anzeigt und von dem einen oder den mehreren CO₂-Sensoren erhalten wird, so dass die O₂-Versorgung gestoppt oder reduziert wird, während der Patient ausatmet.

17. System nach Anspruch 11, worin die Plattformeinheit transportabel ist.

18. System nach Anspruch 11, worin die Kommunikationseinheit weiterhin angepasst ist, Informationen und/oder Daten von der Analyseeinheit kabellos zu empfangen.

19. System nach Anspruch 11, worin die Daten, die den CO₂-Spiegeln entsprechen, eine CO₂-Konzentration, eine CO₂-Wellenform, eine Änderung in der CO₂-Konzentration über die Zeit, endexspiratorisches CO₂ (EtCO₂) oder jegliche Kombination davon umfassen.

20. System nach Anspruch 11, worin der eine oder die mehreren CO₂-Sensoren einen auf Infrarot basierenden CO₂-Sensor, einen auf Halbleiter basierenden CO₂-Sensor, einen auf Nanotechnologie basierenden CO₂-Sensor oder jegliche Kombination davon umfassen.

21. System nach Anspruch 11, worin die entfernte Analyseeinheit eine Prozessoruntereinheit, ein Nutzerinterface, eine Anzeige, eine Kommunikationsuntereinheit oder jegliche Kombination davon umfasst.

## Revendications

1. Un dispositif de surveillance de souffle comprenant :
une unité d'interface de patient adaptée pour être placée sur la tête du patient, ladite unité d'interface de patient comprenant une canule pour collecter un échantillon du souffle exhalé par le patient ; et
une unité de plateforme, dans lequel ladite unité de plateforme comprend :
un ou plusieurs capteurs de dioxyde de carbone (CO2) pour détecter les niveaux de CO2 dans l'échantillon,
une pompe pour transférer l'échantillon de la canule vers un ou plusieurs capteurs de CO2,
et
une unité de communication configurée pour transmettre sans fil les données correspondant aux niveaux de CO2 à une unité d'analyse distante, caractérisé en ce ladite unité de plateforme est configurée pour être attachée au lit ou au siège du patient et pour être physiquement connectée à ladite unité d'interface de patient.

2. Le dispositif selon la revendication 1 dans lequel l'unité de plateforme comprend en outre un centre de contrôle, une ou plusieurs sources de courant, un ou plusieurs adaptateurs, un ou plusieurs capteurs additionnels ou toute combinaison de ceux-ci.

3. Le dispositif selon la revendication 2 dans lequel les un ou plusieurs capteurs additionnels comprennent un capteur de température, un capteur de saturation d'oxygène sanguin, un capteur de pression sanguine, un capteur de rythme cardiaque, un capteur de rythme respiratoire, un capteur d'activité électrique cardiaque, un capteur d'activité cérébrale ou toute combinaison de ceux-ci.

4. Le dispositif selon la revendication 2 dans lequel lesdits capteurs comprennent des adaptateurs pour connexion à un échantillonneur, à la fourniture d'02, à une source de courant ou toute combinaison de ceux-ci.

5. Le dispositif selon la revendication 4 dans lequel ladite unité de plateforme est configurée pour ajuster la fourniture d'02.

6. Le dispositif selon la revendication 5 dans lequel ladite unité de plateforme est configurée pour ajuster la fourniture d'02 basée sur signal indicateur d'inhalation et/ou d'exhalaison reçu à partir desdits un ou plusieurs capteurs de CO2 telle que l'approvisionnement en 02 est arrêté ou réduit durant l'exhalaison du patient.

7. Le dispositif selon la revendication 1 dans lequel l'unité de plateforme est portable.

8. Le dispositif selon la revendication 1 dans lequel l'unité de communication est en outre configurée pour recevoir sans fil des informations et/ou des données à partir de l'unité d'analyse.

9. Le dispositif selon la revendication 1 dans lequel les données correspondant aux niveaux de CO2 comprennent la concentration, une forme d'onde de CO2, un changement en concentration de CO2 au cours du temps, la fin de marée de CO2 (Et CO2) ou toute combinaison de ceux-ci.

10. Le dispositif selon la revendication 1 dans lequel les un ou plusieurs capteurs de CO2 comprennent un capteur de CO2 à infra-rouge, un capteur de CO2 basé sur semi-conducteur, un capteur de CO2 basé sur la nanotechnologie ou toute combinaison de ceux-ci.

11. Un système de surveillance du souffle comprenant :
une unité d'interface de patient adaptée pour être placée sur la tête du patient, ladite unité d'interface de patient comprenant une canule pour collecter un échantillon du souffle exhalé par le patient ;
une unité de plateforme, ladite unité de plateforme comprenant :
un ou plusieurs capteurs de CO2 pour détecter les niveaux de CO2 dans l'échantillon,
une pompe pour transférer l'échantillon de la canule vers un ou plusieurs capteurs de CO2, et
une unité de communication configurée pour transmettre sans fil les données correspondant aux niveaux de CO2 et
une unité d'analyse distante configurée pour recevoir sans fil les données correspondant aux niveaux de CO2 à partir de l'unité de plateforme et pour traiter ensuite et/ou afficher lesdites données ou informations correspondant auxdites données, caractérisé en ce ladite unité de plateforme est configurée pour être attachée au lit ou au siège du patient et pour être physiquement connectée à ladite unité d'interface de patient.

12. Le système selon la revendication 11 dans lequel l'unité de plateforme comprend en outre un centre de contrôle, une ou plusieurs sources de courant, un ou plusieurs adaptateurs, un ou plusieurs capteurs additionnels ou toute combinaison de ceux-ci.

13. Le système selon la revendication 12 dans lequel les un ou plusieurs capteurs comprennent un capteur de température, un capteur de saturation d'oxygène sanguin, un capteur de pression sanguine, un capteur de rythme cardiaque, un capteur de rythme respiratoire, un capteur d'activité électrique cardiaque, un capteur d'activité cérébrale ou toute combinaison de ceux-ci.

14. Le système selon la revendication 13 dans lequel lesdits capteurs comprennent des adaptateurs pour connexion à un échantillonneur, à la fourniture d'02, à une source de courant ou toute combinaison de ceux-ci.

15. Le système selon la revendication 14 dans lequel ladite unité de plateforme est configurée pour ajuster la fourniture d'02.

16. Le système selon la revendication 15 dans lequel ladite unité de plateforme est configurée pour ajuster la fourniture d'02 basée sur un signal indicateur d'inhalation et/ou d'exhalaison reçu à partir desdits un ou plusieurs capteurs de CO2 telle que l'approvisionnement en 02 est arrêté ou réduit durant l'exhalaison du patient.

17. Le système selon la revendication 11 dans lequel l'unité de plateforme est portable.

18. Le système selon la revendication 11 dans l'unité de communication est en outre configurée pour recevoir sans fil des informations et/ou des données à partir de l'unité d'analyse.

19. Le système selon la revendication 11 dans lequel les données correspondant aux niveaux de CO2 comprennent la concentration en CO2, une forme d'onde de CO2, un changement en concentration de CO2 au cours du temps, la fin de marée de CO2 (Et CO2) ou toute combinaison de ceux-ci.

20. Le système selon la revendication 11 dans lequel les un ou plusieurs capteurs de CO2 comprennent un capteur de CO2 à infra-rouge, un capteur de CO2 basé sur semi-conducteur, un capteur de CO2 basé sur la nanotechnologie ou toute combinaison de ceux-ci.

21. Le système selon la revendication 11 dans lequel l'unité d'analyse distante comprend une sous-unité de processeur, une interface d'utilisateur, une sous-unité de communication ou toute combinaison de celles-ci.
